# EUROPEAN PATENT APPLICATION

(11) **EP 1 733 742 A1**
(43) Date of publication of application: **20.12.2006**
(21) Application number: 05076421.6
(22) Date of filing: 17.06.2005
(51) Int. Cl.: A61K 47/48, C08G 83/00

(54) **Dendrimers multivalently substituted with active groups**

(71) Applicant: Universiteit Utrecht Holding B.V., 3584 CL Utrecht (NL)
(72) Inventor: Liskamp, Robertus Matthias Joseph, 3573 XL Utrecht (NL); Rijkers, Dirk Thomas Sigurd, 5612 BA Eindhoven (NL); Pieters, Roelof Jan, 3524 VJ Utrecht (NL); Brouwer, Arwin John, 3581 LP Utrecht (NL); Joosten, Johannes Albertus Frederikus, 6691 EH Gendt (NL)
(74) Representative: Winckels, Johannes Hubertus F.

(57) **Abstract**

The present invention relates to dendrimers being multivalently substituted with active groups, as well as to processes for the preparation thereof and to the use of such multivalent dendrimers.
The active compounds encompass peptides, including oligopeptides up to entire proteins, carbohydrates and pharmaceutically active drug molecules.
Active compounds are chemoselectively bound to the dendrimer periphery using a cycloaddition reaction (also termed as "click" reaction) between an alkyne group and either an azide or a nitrile oxide, leading to a 1,4-disubstituted 1,2,3-triazole or a 1,4-disubstituted 1,2-oxazole bridging unit. Such reaction is catalyzed by Cu(I) compounds. Microwave irradiation improves the efficiency of the click reaction.

## Description

The present invention relates to dendrimers multivalently substituted with active groups, and especially with biologically active groups or with diagnostically usable groups. In addition, the present invention relates to a process for substituting particular dendrimers with said active groups. Further, the present invention relates to intermediates for these multivalent dendrimers. In yet a further aspect, the present invention relates to the use of the multivalent dendrimers of the invention.

In the art, and especially in the pharmaceutical field or in the field of diagnosis, there is a need to have available compounds containing a number of active moieties. One compound containing a number of active moieties and preferably more than two of such active moieties, which preferably are the same active moiety, is called in the present description and appending claims a "multivalent" compound. Multivalent compounds lead to an increase in activity, and especially in biological or systemic activity or diagnostic efficiency. More in particular, multivalency is an important principle in nature to increase weak interactions, e.g. between ligands and receptors, to biologically important levels.

The present invention is especially directed to dendrimers. As the skilled person knows, dendrimers are molecules that at least partially consist of repeating units, and which are molecules that have a two- or three-dimensional diverting branchingstructure starting from the "base" of the dendrimer. The name is derived from the greek word for tree; dendrimers are (often) tree-shaped molecules.

The "base" or "core" of the dendrimer was the starting point for its assembly in the chemical construction. Where the assembly of the dendrimer skeleton ends, the "periphery" or "surface" of the dendrimer, a number of active groups are substituted leading to a multivalent dendrimer.

Dendrimers are molecular scaffolds for increasing effects merely by offering a number of ligands or by aligning these ligands. A crucial issue is the complete and efficient attachment of ligands to dendrimers. In the prior art, there are only a few possibilities of attaching peptides to complex molecules such as dendrimers; and even less possibilities to attach unprotected peptides to complex molecules such as dendrimers, let alone possibilities usable at a reproducible an industrial scale. In most cases peptides are attached to dendrimers by chemoselective reaction of sulfhydryl groups of peptides, and especially of the cysteine moieties thereof, with maleimide or iodoacetamide functionalities. However, new reactions with increased efficiency and chemoselectivity (preferably at physiological conditions) would be very welcome.

Particularly, there is a need for a multivalent display of active compounds, and especially a multivalent display of peptides and carbohydrates, in order to increase the affinity of binding to e.g. bacteria, bacterial toxins, enzymes, reactors, antigens or antibodies, etc.

It is an object of the present invention to provide multivalent dendrimers which are well-defined.

It is another object of the present invention to provide homodisperse multivalent dendrimers.

It is yet another object of the present invention to provide processes to prepare the dendrimers of the objects defined in the previous paragraphs, especially in a convenient and preferably efficient way. This especially to overcome the problems of the prior art methods that vary in efficiency and compatibility with functional groups, and which known methods tend to yield all kinds of side-products which are undesirable in couplings involving very specific interactions.

It is yet another object to provide a process which allows a flexible method of introducing a wide variety of active compounds in the multivalent dendrimers.

The present invention aims to solve at least one, preferably more, most preferably all of the foregoing objects.

In a first aspect, the present invention relates to a multivalent dendrimer, comprising at the dendrimer periphery a number of active compounds, the active compounds being coupled to the dendrimer through a 1,2,3-triazole or 1,2-oxazole aromatic heterocyclic bridge.

The multivalent dendrimers of the invention are versatile constructs for the simultaneous presentation of especially biologically relevant ligands. These multivalent constructs are especially interesting for enhancing the interaction of weakly interacting individual ligands e.g. carbohydrates.

This aromatic heterocyclic bridge may consist only of the aromatic heterocyclic linking group, but may additionally contain one or more spacer groups.

The nature of the spacer or linking group is not critical, as long as it does not adversely interfere with the intended end use of the final multivalent dendrimers of the invention. Actually, the use of spacer groups may have an advantage in that the active compounds, for instance compounds that are recognized by receptors in a system such as an animal or human body, have more flexibility to be able to bind to one or more of such receptors without being hindered by the dendrimeric starting structure. Examples of suitable spacers are for instance hydrophilic spacers, such as those consisting of straight or branched C₁₋₆ alkylene groups, such as ethylene or propylene moieties arranged between two hydrophilic groups such as oxo, thio, amino, amide or ureido groups.

Although it is possible to achieve some of the objects of the present invention, when the dendrimer only contains two or three branches (a first generation dendrimer), it is preferred that the dendrimer is at least a second generation dendrimer, preferably at least a third or fourth generation dendrimer. A second generation dendrimer is a dendrimer wherein the branches derived from the repeating unit (or monomer) defining the dendrimer are coupled (optionally through a linking group or a spacer) to a further repeating unit. For the third and further generation these further repeating units are subsequently coupled to further repeating units.

The number of active compounds coupled to the periphery of the starting dendrimer is at least 2, but preferably higher. Particularly, multivalent dendrimers encompass, but are not limited to, di-, tri-, tetra-, octa-, nona- and hexadecavalent dendrimers. By combining two or more monomers, almost all possible valencies are achievable. For instance, if a monomer having two arms and a monomer having three arms are attached to a monomer having two arms, a pentavalent system is obtained. Starting from and using only monomers having three arms a second generation will have 9, a third generation will have 27 and a fourth generation will have 81 valencies.

Many dendrimers known in the prior art are heterodisperse, because they are mixtures of different molecules. Particularly, during their formation defects occur, because in the respective syntheses, it is very difficult to achieve complete reactions especially in higher generations.

These known heterodisperse dendrimers can form the basis for the multivalent dendrimers according to the invention. However, in accordance with the present invention also homodisperse multivalent dendrimers may be provided, these homodisperse multivalent dendrimers forming a preferred embodiment of the present invention. In a further preferred embodiment, the dendrimer forming the basis of the presently described multivalent dendrimers is amino acid based. Such amino acid based dendrimers provide the possibility of being homodisperse, and in addition are easily preparable.

Amino acid based dendrimers are known in the art and can for instance be synthesized by the method described by Brouwer et al. in Eur. J. Org. Chem. (2001), 1903-1915. Particularly, a reliable and efficient method is described in said reference using amino acids as repeating unit, which amino acids contain at least two amino groups and one carboxyl acid group or at least two carboxylic acid groups and one amino group. The remainder of these amino acids is not critical, as long as the reaction between the amino and the carboxylic acid groups is not disturbed. Particularly, dendrimers can be composed of natural aminoacids, for example those present in proteins. Suitable examples are lysine, diaminobutyric acid, and diaminopropionic acid. However, also other (synthetic) amino acids can be used. Amino acids useful in the dendrimers of the present invention may e.g. comprise an aromatic nucleus providing rigidity or pre-organisation to the ultimate dendrimer, which is believed beneficial for its activity. An example of such a monomer is 3,5-di-(2-aminoethoxy)-benzoic acid. However, also the other monomers taught in said Brouwer *et al.* reference can be suitably used.

In the multivalent dendrimer according to the invention, each active compound coupled to the periphery of the dendrimer is preferably selected from the group consisting of peptides, carbohydrates or drugs.

Especially when the active compounds are the same, that is, represent the same group, very useful dendrimers are obtained. This, however, does not mean that when the active compounds do not have the same meaning, a less suitable multivalent dendrimer is obtained. If, for instance a number of different peptide fragments of proteins expressed by disease causing, or otherwise undesired bacteria or viruses are attached to the dendrimer of the invention, a synthetic vaccine or an antibacterial or antiviral composition may be created.

In a further preferred embodiment, the multivalent dendrimer according to the invention, comprises a core that is substituted by another active compound than the active compounds at the periphery.

The multivalent dendrimers of the present invention will be described in more detail, herein-below.

In a second major aspect, the invention relates to a process for the preparation of the multivalent dendrimers according to the invention, which process comprises reacting a dendrimer having a number of alkyne moieties at its periphery with active compounds comprising an azide, nitrile oxide or nitrone moiety, or reacting a dendrimer having a number of azide or nitrile moieties at its periphery with active compounds comprising an alkyne moiety.

One of the major advantages of the present invention is that the core of the dendrimer is available for a substitution different from the active compounds. A further important aspect is that by using suitable dendrimer building units the exact number of valencies can be controlled in such a way that optimal (binding) activity can be achieved for the multivalent active groups.

This process is based on the cycloaddition reaction between an acetylene and either an azide or an nitrile oxide, leading to a 1,4-disubstituted 1,2,3-triazole or a 1,4-disubstituted 1,2-oxazole bridging unit. This cycloaddition reaction, especially leading to an 1,2,3-triazole, is also referred to as the "click" reaction. The click reaction between the azide and acetylene is known as the Huisgen cycloaddition and is catalyzed by Cu(I) compounds.

The present inventors found that this click reaction is very suitable for chemoselective reactions on dendrimers, and especially with an aim of introducing multivalent peptide and carbohydrate active groups.

Recently, this click reaction, also in combination with microwave, has been described for a number of different reactions. See for instance Savin et al. in Molecular Diversity 7 (2003), 171-174. However, this reaction has not been described to prepare multivalent dendrimers wherein the potential click sites are completely converted to 1,2,3-triazole or 1,2-oxazole bridges. Click reactions known to the present inventors generally are used to form one single bridge, or alternatively for non-complex reaction systems.

Especially when the reaction is microwave assisted, a very effective and efficient preparation process is obtained. The use of a microwave results in an efficient and short heating, which heating step is especially effective in the coupling of peptides and carbohydrates to the dendrimer. It effects a very high, and even complete conversion of the alkyne or alternatively the azide or nitrile oxide moieties present at the periphery of the dendrimer. This results in a defined and reproducible multivalent dendrimer product, which is essential to prepare such multivalent dendrimers for pharmaceutical and diagnostical purposes on a practically useful scale.

The microwave assisted click reaction is so quick and selective, that in many cases the active compound or group to be coupled to the periphery of the dendrimer need not be protected. When active groups, such as peptides and carbohydrates, are used in an unprotected form, a deprotecting step is not required. This results in a much simpler process. No interference occurs with other groups than the essential groups for the click reaction.

Because the ― preferably- amino acid based dendrimers having a number of alkyne moieties at its periphery, or having a number of azide or nitrile oxide moieties at its periphery have, for as far as the inventors know, not been described in the prior art, these intermediates in the process of preparing the multivalent dendrimers of the invention also form part of this invention.

Yet another aspect of the invention relates to the use of the multivalent dendrimers described in the present description or made by the process described herein, as a pharmaceutical agent or as a diagnostic agent.

Now, the invention will be described in more detail.

As said, the dendrimers used as starting compound in the present invention contain particular surface groups which are able to form a covalent bond via cycloaddition to the active group forming moieties. Particularly, the cycloaddition reaction used in the present invention leads to the creation of 1,2,3-triazole or 1,2-oxazole aromatic heterocyclic bridges between the dendrimer and the active compound. Such bridges are formed by the click reaction between an alkyne and an azide or between an alkyne and on nitrile oxide moiety. It is possible to have the alkyne or acetylene moiety present as a substituent attached to the active compound and the azide or nitrile oxide moiety present as substituent of the dendrimer; it is however preferred to have the alkyne or acetylene moiety present as substituent on the dendrimer, and the azide or nitrile oxide group as substituent attached to the active compound.

The dendrimers used as starting compound in the present invention contain alkyne, azide or nitrile oxide moieties at the periphery or surface. These moieties can be introduced after completion of the (known) dendrimer synthesis or alternatively by incorporation as part of the last peripheral or surface-dendrimer monomer used for construction of the dendrimer.

Preferably, the dendrimers used are amino acid based dendrimers. One of the reasons as to why the amino acid based dendrimers are preferred is that amino groups can easily be converted in azido groups or alternatively acetylene/alkyne groups can be attached. The former is preferred when the active compound to be attached has an acetylene or alkyne substitution. Moreover, azide or acetylene group containing dendrimer building units can easily be synthesized.

The same reason that an amino group can be easily converted in an azido group, makes that all sorts of peptides, ranging from oligopeptides up to entire proteins, can be clicked to dendrimers containing acetylene groups at their periphery. Not only can the N-terminus of each peptide serve as a "handle" for the click reaction, but also each amino acid residue containing an amino group in the side chain, such as a lysine residue or such a side group in synthetic amino acids. Alternatively, azido function containing "amino" acids, or other azide containing auxiliaries can be introduced in for instance a peptide-type active group. Also-SH groups present in, for instance, peptide structures can be used to introduce, for example, azide groups.

Dendrimers of the invention containing at their periphery a high number of groups able to form the aromatic heterocyclic bridges in a click reaction can even be used to attach unprotected peptides.

Many azide containing compounds inclusive dendrimers can be prepared by converting an amino moiety into an azide moiety even when other reactive groups are present. In the working examples herein-below an example of such a conversion is shown. Further, examples of azido (di)peptides and biologically relevant azido peptides, such as Leu-enkephalin, antimicrobial peptides, which can easily be converted into the cycloadducts of the present invention will be shown.

The skilled person also knows that acetylene moieties can easily be introduced in particular molecules. Also for this introduction, examples will be given herein-below.

Densely functionalized dendrimers are promising protein mimics.

The process of the present invention, especially when microwave-assisted, leads to an increased efficiency, is very fast and can be used for a variety of active compounds containing an acetylene, azide or nitrile oxide moiety. The click reaction is catalysed by copper catalysts, and especially copper compounds soluble in the reaction solvent.

The active groups to be used to form the multivalently substituted dendrimers of the invention encompass peptides, including oligopeptides up to entire proteins, carbohydrates and pharmaceutically active drug molecules. Such active groups, and especially multivalent dendrimeric peptides according to the invention can, for instance, be used as synthetic vaccines, for diagnostic purposes or as targeting devices, for example in the treatment of cancer or infections.

In one of the most preferred embodiments, also the "base" of the dendrimer can be substituted, either by an active compound, such as a homing device or targeting moiety, e.g. a peptide, protein or antibody; or by a label, such as a fluorescent label; by an isotope chelator; by a drug; or by an adjuvant. More in detail, at the "base" or "core" of the dendrimer, a functional group may be present that can be used for attachment of a functional group.

By a suitable combination of substitutions at the periphery and at the base, the present invention renders it possible to achieve a wide variety of applications.

For example, if the base of the dendrimer contains a fluorescent label or a chelator, and the periphery contains a number of peptides or carbohydrates, valuable diagnotics or imaging reagents can be provided. If for instance a radio isotope is present, this may be used for selective *in vivo* irradiation; if a stable isotope is present, this may be used for *in vivo* MRI, *etc.* Also a drug compound can be targeted to a particular site *in vivo* to achieve a very selective application of the drug, which is, e.g., highly suitable to treat certain types of cancer. In these embodiments, the substituted periphery of the dendrimer effects the desired targeting.

Alternatively, it is also possible to have the targeting moiety, such as an antibody, attached to the base of the dendrimer to escort a dendrimer containing a high number of, e.g., anti-cancer drugs to a selected site in a system.

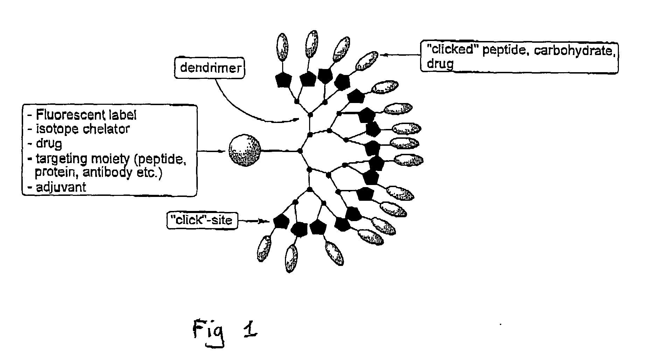

Figure 1 shows a schematic representation of a multivalent dendrimer according to the invention.

The present invention will now be described in more detail, while referring to the following, non-limiting examples. Unless otherwise indicated, percentages are percentages by weight drawn to the weight of the total product.

In these examples, the design, synthesis and biological evaluation of dendrimeric carbohydrates will be shown. More in detail, it will be shown that peptides can be efficiently attached to a derivatized version of the earlier described (see the article of Brouwer *et al.* referred to herein-above) dendrimers using a 1 ,3-dipolar cycloaddition ("click"-chemistry), which was conveniently assisted by microwave irradiation to ensure a complete modification of the alkyne end groups.

The synthesis will be described of the required dendrimeric alkynes used in the attachment of azido peptides either derived from the α-amino group, ε-amino group of lysine or an ω-aminohexanoyl spacer to obtain di-, tetra-, octa- and hexadecavalent dendrimeric peptides.

### Introduction to the Examples

The peptides used in the examples were synthesized on an Applied Biosystems 433 A Peptide Synthesizer.

Analytical HPLC runs were carried out on a Shimadzu HPLC system and preprative HPLC runs were performed on a Gilson HPLC workstation. Analytical HPLC runs were performed on Alltech Adsorbosphere XL C18 and Alltech Prosphere C4 columns (250 x 4.6 mm, pore size 300 Å, particle size: 5 µm) or on a Merck LiChroCART CN column (250 x 4.6 mm, pore size 100 Å, particle size 5 µm) at a flow rate of 1.0 mL/min using a linear gradient of buffer B (0-100% in 25 min) in buffer A (buffer A: 0.1% TFA in H₂O buffer B: 0.1% TFA in CH₃CN/H₂O 95:5 v/v). Preparative HPLC runs were performed on an Alltech Prosphere C4 column (250 x 22 mm, pore size 300 Å, particle size 10 µm) or on a Merck LiChroCART CN column (250 x 10 mm, pore size 100 Å, particle size: 10 µm) at a flow rate of 4.0 mL/min using a linear gradient of buffer B (0.100% in 50 min) in buffer A (buffer A: 0.1% TFA in H₂O buffer B: 0.1 TFA in CH₃CN/H₂O 95:5 v/v.

Liquid chromatography electrospray ionization mass spectrometry was measured on a Shimadzu LCMS-QP8000 single quadrupole bench-top mass spectrometer operating in a positive ionization mode.

MALDI-TOF analysis was performed on a Kratos Axima CRF apparatus with bradykinin(1-7) monoisotopic [M + H]⁺ 757.399), human ACTH(18-39) (monoisotopic [M + H]⁺2465.198) bovine insulin oxidized B chain (monoisotopic [M + H]⁺ 3494.651), bovine insulin (monoisotopic [M + H]⁺ 5730.609) and equine cytochrome c (average [M + H]⁺ 12361.96) as external references and α-cyano-4-hydroxycinnamic acid or sinapic acid as matrices.

¹H-NMR spectra were recorded on a Varian G-300 (300 MHz) spectrometer and chemical shifts are given in ppm (δ) relative to TMS. ¹³C-NMR spectra were recorded on a Varian G-300 (75,5 MHz) spectrometer and chemical shifts are given in ppm (δ) relative to CDCl₃ (77.0 ppm). The ¹³C-NMR spectra were recorded using the attached proton test (APT) sequence. *R*_{f} values were determined by thin layer chromatography (TLC) on Merck precoated silicagel 60F254 plates. Spots were visualized by UV-quenching, ninhydrin or Cl₂/TDM¹. Elemental analyses were done by Kolbe Microanalytisches Labor (Mülheim an der Ruhr, Germany).

The general procedure for the microwave-assisted click reaction: the alkyne (1 equiv.) and the azide (1.3 equiv. per arm) were dissolved in 3 mL DMF/H₂O 1:1 v/v. To this solution, CuSO₄.5H₂O (0.05 equiv.) and Na-ascorbate (0.50 equiv.) were added. The reaction mixture was placed in a microwave reactor (Biotage) and irradiated during 5-30 min. at 100 °C. The cycloaddition reaction was monitored on TLC for completion of the reaction.

### Example 1

The approach described by Brouwer *et al.* (*ibid.*) for the convergent synthesis of amino acid based dendrimers was adapted in order to obtain dendrimers with surface propargyl groups to enable a 1,3-dipolar cycloaddition ("click") reaction with amino acid /peptide derived azides.

First generation dendrimer 1 was synthesized from 3,5-dihydroxybenzoic acid in an overall yield of 81% as shown in Scheme 1. In detail, 3,5-dihydroxymethylbenzoate (21.4 g, 130 mmol) was dissolved in dry DMF (250 mL) and anhydrous K₂CO₃ (45 g, 330 mmol, 2.5 equiv.) was added. To this suspension, a solution of propargylbromide in toluene (35 mL, 314 mmol, 2.5 equiv.) was added dropwise. The reaction mixture was stirred for 48 h at room temperature. Then, DMF was removed by evaporation and the residue was redissolved in EtOAc (400 mL) and the organic phase was washed with H₂O (3 x 100 mL) in KHSO₄ (3 x 100 mL) and brine (3 x 100 mL), dried (Na₂SO₄) and *evaporated in vacuo.* The residue was recrystallized from EtOAc/hexane to obtain 1 as off-white crystals in 81% yield (25.2 g). *R*_{f}(EtOAc/hexane 4:1 v/v): 0.76; *R*_{f} (DMC/MeOH 98:2 v/v): 0.87; *R*_{f}(CHCl₃/MeOH/AcOH 95:20:3 v/v): 0.83; ¹H-NMR (CDCl₃) δ 2.55 (t (*J* 2.47 Hz), 2H), 3.91 (s, 3H), 4.72 (d (*J* 2.47 Hz), 4H), 6.81 (t (*J* 2.20 Hz), 1H), 7.29 (d (*J* 2.20 Hz), 2H); ¹³C-NMR (CDCl₃) δ 52.4, 56.0, 76.0, 77.9, 107.5, 108.8, 132.0, 157.8, 158.4; Elemental analysis:
calculated for C₁₄H₁₂O₄ C 68.83, H 4.95, found C 68.76, H 4.95.

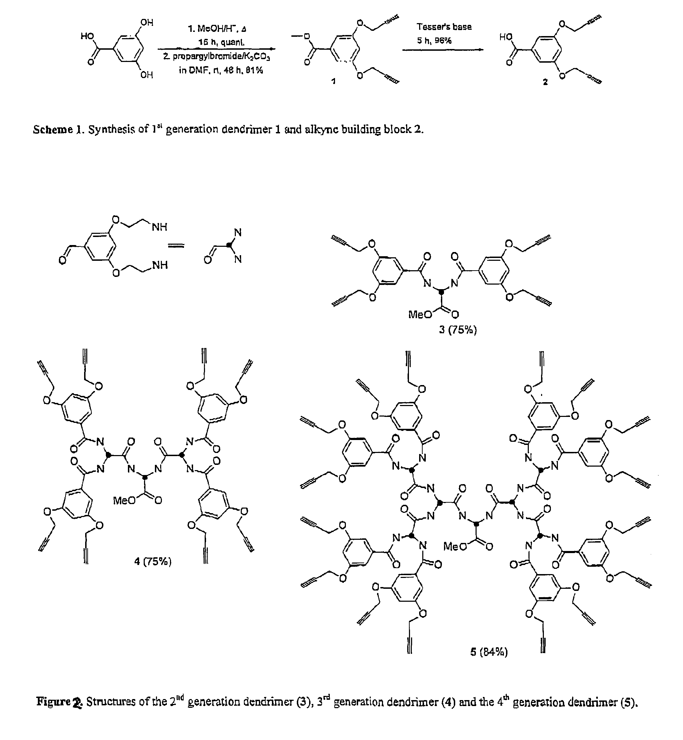

Methyl ester 1 was saponified with Tesser's base (Tesser and Balvert-Geerts, Int. J. Peptide Protein Res. 7, (1975), 295) to obtain acid 2 and was subsequently used in the synthesis of the second , third and fourth generation dendrimer **3 , 4** and **5 ,** respectively in 75-84 % yields (Figure 2).

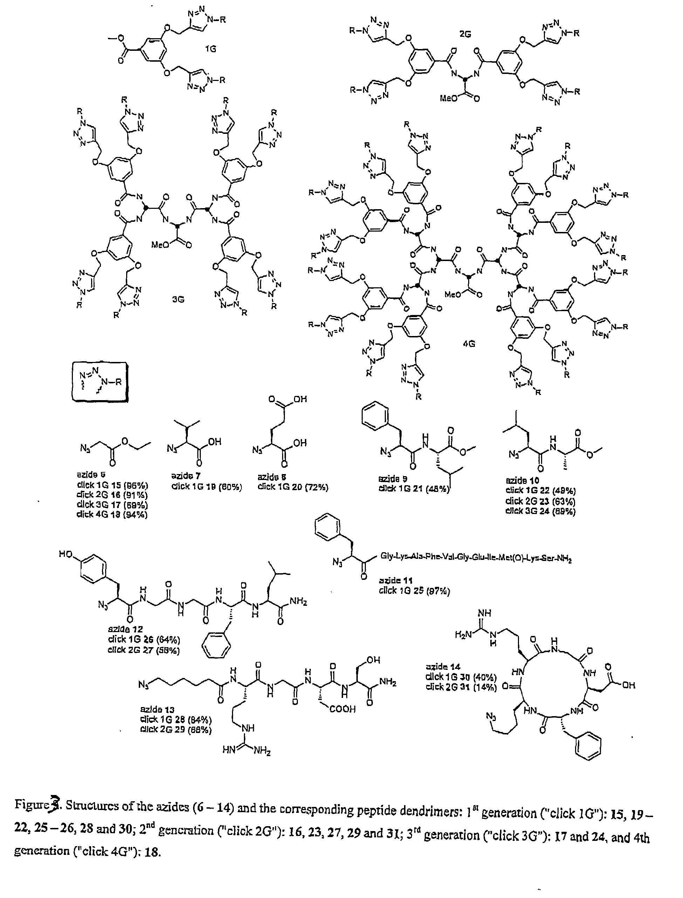

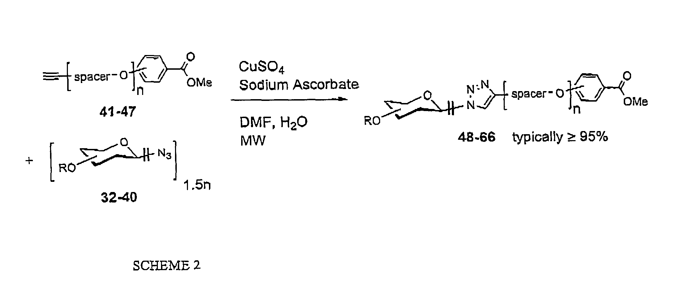

The azido peptides **6-14** used were synthesized according to literature procedures (Azide **6** was prepared according to: S.G. Alvarez and M.T. Alvarez, Synthesis, (1997), 413; Azides **7-10, 13** and **14** were synthesized according to: J.T. Lundquist, IV and J.C. Pelletier, Org. Lett. 3, (2001), 781; azido peptides **11** and **12** were synthesized according to: D.T.S. Rijkers et al., Tetrahedron Lett., 43 (2002), 3657) (Figure 3).

Azide **6** was mixed with acetylene **1** in the presence of CuSO₄ /Na-ascorbate/Cu-wire in different solvent systems for 16 h at room temperature. Monitoring by TLC showed that formation of the monovalent cycloadduct proceeded rapidly, and conversion to the divalent product w s sluggish. Despite this 15 was isolated in fair yields (43-56%).

### Example 2

The results of Example 1 were tremendously improved by running the reaction under microwave irradiation. After 10 min at 100°C using THF/H₂O (1/1) as a solvent in the presence of CuSO₄/Na-ascorbate, **15** was isolated in 93 % yield (Figure 3). A slight further improvement of the yield to 96% w as achieved by performing the reaction in aqueous DMF.

Using the former solvent systems, divalent peptide cycloadducts **19-22** (Figure 3) were obtained in fair to good yields (48-72 %).

The lower yields may be explained by the (significant) lower solubility o f **19-22** compared to **15.**

### Example 3

Amino acid azide **6** and peptide azide **10,** respectively, were reacted with second generation dendrimer **3** for 5-10 min at 100°C. Amino acid azide **6** gave a precipitate which was only soluble in DMF or DMSO and was characterized (¹H-, ¹³C-NMR (APT, HMBC and HSQC), ESMS and MALDI-TOF) as tetravalent amino acid cycloadduct **16** and obtained in excellent yield (91 %).

In detail, for **16:** alkyne **3** (57 mg, 84 µmol, **1** equiv) and azide **6** (70 mg, 545 µmol, 1.6 equiv per arm) were dissolved in THF/H₂O (2 mL, 1/1) and Na-ascorbate (8 mg, 40 µmol, 50 mol-%) followed by CuSO₄.5H₂O (1 mg, 4 µmol, 5 mol-%) were added. The reaction mixture was placed in the microwave reactor (Biotage) and irradiated at 100°C during 5 min. The precipitate was filtered off, washed with ice-cold methanol and dried in a dessicator. Compound 16 was obtained as a white solid in 91% yield. *R*_{f}(CHCl₃/MeOH/AcOH 95/20/3): 0.68; ¹H-NMR (300 MHz, DMSO-d₆): δ 1.21 (t, 12H, J = 7.14 Hz), 3.59 (m, 4H), 3.81 (s, 3H), 4.17 (q, 8H, J = 7.14 Hz), 4.21 (m, 4H), 5.21 (s, 8H), 5.42 (s, 8H), 6.82 (m, 1H), 6.91 (m, 2H), 7.09 (m, 2H), 7.15 (m, 4H), 8.24 (s, 4H), 8.63 (t, 2H); 13C-NMR (75 MHz, DMSO-d₆): δ 14.2, 40.5, 50.6, 52.5, 61.5, 61.7, 66.6, 104.5, 106.6, 107.8, 126.3, 131.8, 136.5, 142.7, 159.2, 159.9, 166.1, 167.4; MALDI-TOF: calcd for C₅₄H₆₂N₁₄O₁₈: 1194.437, found: [*M* + H]⁺ 1195.597, [*M* + Na]⁺ 1217.578; elemental analysis calcd for C₅₄H₆₂N₁₄O₁₈: C 54.27%, H 5.23%, N 16.41%, found: C 54.16%, H 5.17%, N 16.22%.

Tetravalent peptide cycloadduct **23** was isolated by extraction with EtOAc and obtained in a fair yield of 63% after crystallization from EtOAc/hexane.

Using microwave irradation, octavalent peptide **24** (48 %) as well as octavalent and hexadecavalent systems **17** and **18** were prepared in 69 and 94 % yield, respectively (Figure 3).

### Example 4

In this example the attachment of larger or bioactive peptides to these dendrimeric systems using the microwave-assisted cycloaddition chemistry is described. Particularly, azido peptide **11,** representing the amino acid residues 12-23 of the antimicrobial peptide magainin I amide (Zasloff, Proc. Natl. Acad. Sc. USA 84 (1987), 5449) was coupled under microwave assistance and divalent cycloadduct **25** was obtained in nearly quantitative yield (Figure 3). Work-up of this water-soluble divalent peptide was easy as well as purification by HPLC or size exclusion chromatography.

Then, azido-Leu-enkephalin **(12),** azide **13** (a fibronectin active fragment, connected to an ω-amino hexanoic acid spacer) and the cyclo RGD azido peptide **14** (an ανβ₃ integrin binding RGD peptide for tumor targeting; Haubner et al., J. Am. Chem. Soc., 18 (1996), 7461; Haubner et al., J. Nucl Med. 42, (2001), 326; Janssen et al., Cancer Res., 62 (2002), 6146; Thurmshirn et al., Chem.Eur. J. 9, (2003), 2717) were coupled to alkynes **2** and **3.** The divalent and tetravalent peptides **26, 28, 30** and **27, 29, 31,** respectively (Figure 3) were obtained after purification by HPLC in yields ranging from **14** to 84%. These peptides were identified by MALDI-TOF analysis.

In addition, the coupling of 4^{th} generation dendrimer (16 end-groups) 5 with cyclo RGD azido peptide **14** is illustrated in order to obtain a hexadecavalent peptide dendrimer. HPLC analysis after work-up showed that the starting materials were consumed. By SDS-PAGE, bands were visible corresponding to a molecular weight ranging between 53000-65000 Da, corresponding to an aggregate in a tetra- or pentamer.
For the compounds identified in Examples 1-4, the following analytical data have been obtained:
Compound **2:** ¹H-NMR (DMSO-d₆) δ 2.50 (broad s, 2H), 4.85 (d (*J* 2.20 Hz), 4H), 6.86 (t (J 2.47 Hz), 1H), 7.17 (d (J 2.47 Hz), 2H).
Compound **3:** *R*_{f}(EtOAc/hexane 4:1 v/v): 0.03; *R*_{f}(CHCl₃/MeOH/AcOH 95:20:3 v/v): 0.80; ¹H-NMR (CDCl₃) δ 2.55 (t (*J* 2.47 Hz), 4H), 3.83 (m, 4H), 3.88 (s, 3H), 4.07 (t (J 4.94 Hz), 4H) 4.68 (d (J 2.47 Hz), 8H), 6.54 (t (J 2.20 Hz), 1H), 6.72 (t (J 2.20 Hz), 2H), 6.93 (t (J 5.77 Hz), 2H), 7.05 (d (J 2.47 Hz), 4H), 7.09 (d (*J* 2.47 Hz), 2 H); ¹³C-NMR (CDCl₃) δ 40.4, 53.2, 56.9, 57.6, 77.0, 78.8, 106.3, 107.1, 107.6, 109.0, 132.9, 137.4, 159.6, 160.3, 167.4, 168.1; MS analysis:
   calculated for C₃₈H₃₄N₂O₁₀ 678.22, found ES-MS 679.40 [M + H]⁺, 701.45
   [M + Na]⁺; MALDI-TOF 679.298 [M + Na]⁺.
Compound **4:** *R*_{f}(CHCl₃/MeOH/AcOH 95:20:3 v/v): 0.73; ¹H-NMR (DMSO-d₆) δ 2.50 (broad s, 8H), 3.59 (m, 12H) 3.61 (s. 3H)m 4.14 (m, 12 H)m 4.83 (d, 8H), 6.78 (m, 7H), 7.12 (m, 14H), 8.68 (m, 6H); ¹³C-NMR (DMSO-d₆) δ 37.8, 49.8, 53.3, 63.7, 76.0, 76.4, 102.5 103.4, 104.2, 105.1, 129.1, 133.8, 155.7, 156.9, 157.1, 163.3; MS analysis: calculated for C₈₆H₇₈N₆O₂₂ 1546.52, found MALDI-TOF 1547.490 [M + H]⁺, 1569.496 [M + Na]⁺.
Compound **5:** ¹H-NMR (DMSO-d₆) δ 2.50 (broad s, 16H), 3.58 (m, 28H), 3.81 (s, 3H), 4.13 (m, 28H), 4.82 (d, 32H), 6.88 (m, 15H), 7.11 (m, 30H), 8.70 (m, 14H):
   MS analysis: calculated for C₁₈₂H₁₆₆N₁₄O₄₆ 3282.33, found MALDI-TOF 3321.467 [M + K]⁺.
Compound **6:** ¹H-NMR (CDCl₃) δ 1.32 (t (J 7.14 Hz), 3H), 3.88 (s, 2H), 4.26 (q (J 7.14 Hz), 2H); ¹³C-NMR (CDCl₃) δ 14.0, 50.2, 61.8, 168.2.
Compound **7:** ¹H-NMR (CDCl₃) δ 1.01/1.03-1.06/1,08 (dd J 15 11 Hz, J 6.88 Hz), 6H), 2.23 (m, 1H), 3.79 (d J 5 49 Hz), 1H.
Compound **8:** ¹H-NMR (CDCl₃) δ 2.09/2.23 (double m, 2 x 1H), 2.59 (m, 2H), 4.13 (m, 1H).
Compound **9:** ¹H-NMR (CDCl₃) δ 0.85/0.88 (dd *J* 6.59 Hz, *J* 1.10 Hz), 6H), 1.26-1.57 (broad m, 3H), 3.02/3.05-3.07/3.09 (dd *J* 14.1 Hz, *J* 7.5 Hz), 1H), 3.28/3.32/3.34 (dd *J* 14.1 Hz, *J* 4.2 Hz), 1H), 3.73 (s, 3H), 4.28 (m, 1H), 4.52 (m, 1H), 6.21 (broad s, 1H), 6.65 (d *J* 8.52 Hz), 1H), 7.29 (m, 5H); ¹³C-NMR (CDCl₃) δ 22.0, 23.0, 24.8, 38.5, 41.5, 50.8, 52.6, 65.5, 127.5, 128.8, 129.8, 136.1, 168.4, 173.1.
Compound **10:** ¹H-NMR (CDCl₃) δ 0.96/0.97-0.98/0.99 (dd *J* 6.4 Hz, *J* 2.3 Hz), 6H), 1.42 (d *J* 7.14 Hz), 3H),, 1.67-1.85 (broad m, 3H), 3.78 (s, 3H), 3.97 (m, 1H), 4.57 (m, 1H), 6.89 (d, 1H).
Compound **11:** Synthesized as described, see reference 3: [M + H]⁺; calculated 1338.70, found 1338.72 (ES-MS).
Compound **12:** *R*ₜ; 17.88 min (C4);Rt: 19.66 min (C18); MS analysis: calculated for C₂₈H₃₆N₈O₆ 580.27 , found ES-MS 581.55 [M + H]⁺, 603.55 [M + Na]⁺.
Compound **13:** *R*ₜ: 12.89 min (C4); *R*ₜ: 15.57 min (C18); MS analysis: calculated for C₂₁H₃₇N₁₁O₈ 571.28, found 572.55 [M + H]⁺.
Compound **14:** *R*ₜ: 16.81 min (C4); MS analysis; calculated for C₂₇H₃₉N₁₁O₇ 629.30 found 630.55 [M + H]⁺, 652.70 [M + Na]⁺, 668.25 [M + K]⁺.
Compound **15:** *R*_{f}:(CHCl₃/MeOH/AcOH 95:20:3 v/v): 0.73; ¹H-NMR (CDCl₃) δ 1.28 (t J 7,14 Hz), 6H), 3.89 (s, 3H), 4.24 (q J 7.14 Hz), 4H), 5.19 (s, 4H), 5.19 (s, 4H), 5.21 (s, 4H), 6.81 (m, 1H), 7.27 (m, 2H), 7.81 (s, 2H); ¹³C-NMR (CDCl₃) δ 14.0, 33.8, 50.8, 52.3, 62.0, 62.4, 106.9, 108.6, 124.3, 132.1, 143.9, 159.1, 166.6; Ms analysis: calculated for C₂₂H₂₆N₆O₈ 502.48 found ES-MS 503.30 [M + H]⁺, 525.30 [M + Na]⁺; MALDI-TOF 503.259 [M + H]⁺.
Compound **16:** *R*_{f}: (CHCl₃/MeOH/AcOH 95:20:3 v/v); 0.68 ¹H-NMR (DMSO-d₆) δ 1.21 (t *J* 7.14 Hz), 12H), 3.59 (m, 4H), 3.81 (s, 3H), 4.17 (q *J* 7.13 Hz), 8H), 4.21 (m, 4H), 5.21 (s, 8H), 5.42 (s, 8H), 6.82 (m, 1H), 6.91 (m, 2H), 7.09 (m, 2H), 7.15 (m, 4H), 8.24 (s, 4H), 8.63 (t, 2H); ¹³C-NMR (DMSO-d₆) δ 14.2, 40.5, 50.6, 52.5, 61.5, 61.7, 66.6, 104.5, 106.6, 107.8, 126.3, 131.8, 136.5, 142.7, 159.2, 159.9, 166.1, 167.4; MS analysis:
   calculated for C₅₄H₆₂N₁₄O₁₈ 1194.437, found MALDI-TOF 1195.597 [M + H]⁺, 1217.578 [M + Na]⁺; Elemental analysis: calculated for C₅₄H₆₂N₁₄O₁₈ C 54.27%, H 5.25%, N 16.41%, found C 54.16%, H 5.17%, N 16.22%.
Compound **17:** ¹H-NMR (DMSO-d₆) δ 1.20 (t J 7.14 Hz), 24H), 3.60 (broad , 12H), 3.79 (s, 3H), 4.18 (m, 28H), 5.21 (s. 16H), 5.41 (s, 16H), 6.72 (m, 2H), 6.81 (m, 1H), 6.93 (m, 4H), 7.04 (m, 6H), 7.18 (m, 8H), 8.23 (s, 8H), 8.68 (m, 6H), MS analysis: calculated for C₁₁₈H₁₃₄N₃₀O₃₈ 2580.50, MALDI-TOF 2581.012 [M + H]⁺, 2603.116 [M + Na]⁺;
   Elemental analysis: calculated for C₁₁₈H₁₃₄N₃₀O₃₈ C 54.8%, H 5.37%, N 16.00%, found C 54.88%, H 5.17%, N 16.19%.
Compound **18:** MS analysis: calculated for C₂₄₆H₂₇₈N₆₂O₇₈ 5347.969, found MALDI-TOF 5389.460 [M +CH₃CN) + H]⁺.
Compound **19:** *R*ₜ: 19.05 min (C4) *R*ₜ: 20.84 min (C18); ¹H-NMR (DMSO-d₆) δ 0.74 (d J 6.59 Hz), 6H), 0.94 (d J 6.59 Hz), 6H), 1.24 (m, 2H), 3.8 4 (s., 3H), 4.10 (broad s, 2H), 5.03 (m, 2H), 5.21 (s, 4H), 7.07 (m, 1H), 7.18 (m, 2H), 8.30 (s, 2H); ¹³C-NMR (DMSO-d₆) δ 18.5, 19.4, 31.0, 52.6, 61.8, 107.0, 108.3, 124.9, 131.8, 134.2, 142.2, 159.4, 166.1, 170.3; MS analysis: calculated for C₁₆H₃₀N₆O₈ 530.21, found MALDI-TOF 531.339
   [M + H]⁺, 553.308 [M + Na]⁺.
Compound **20:** *R*ₜ: 15.98 min (CN); MS analysis: calculated for C₂₄H₂₆N₆O₁₂ 590.16, found ES-MS 591.29 [M + H]⁺.
Compound **21:** ¹H-NMR (CDCl₃) δ 0.85 (d *J* 5.49 Hz), 12H), 1.55 (m, 6H), 3.38 (m, 2H), 3.57 (m, 2H), 3.67 (s, 6H), 3.90 (s, 3H), 4.49 (m, 2H), 5.13 (s, 4H), 5.57 (m, 2H), 6.77 (m, 1H), 6.99 (m, 4H), 7.27 (m, 5H), 7.33 (m, 5H), 7.86 (s, 2H); ¹³C-NMR (CDCl₃) δ 21.6, 22.5, 24.6, 39.5, 40.8, 51.1, 52.2, 61.9, 65.4, 106.8, 108.4, 123.7, 127.2, 128.5, 128.7, 132.0, 134.9, 143.4, 159.0, 166.2, 167.2, 172.3; MS analysis: calculated for C₄₆H₅₆N₈O₁₀ 880, found ES-MS 881.50 [M + H]⁺, 903.30 [M + Na]⁺; MALDI-TOF 881.288 [M + H]⁺, 903.244 [M + Na]⁺, Elemental analysis: calculated for C₄₆H₅₆N₈O₁₀ C 62.71%, H 6.41%; N 12.72%, found C 62.64%, H 6.37%, N 12,64%.
Compound **22:** ¹H-NMR (DMSO-d₆) δ 0.89 (m, 12H), 1.24 (m, 2H), 1.30 (d *J* 7.42 Hz), 6H), 1.89 (broad m, 4H) 3.64 (s, 6H), 3.84 (s, 3H), 4.25 (m, 2H), 5.19 (s, 4H), 5.47 (m, 2H) 7.05 (m, 1H), 7.19 (m, 2H), 8.35 (s, 2H), 9.03 (d J 6.59 Hz), 2H), ¹³C-NMR (DMSO-d₆) δ 17.3, 22.2, 23.0, 24.8, 41.3, 48.5, 52.7, 53.0, 61.4, 62.2, 107.4, 108.7, 132.3, 143.0, 159.9, 166.5, 168.7, 173.3; MS analysis: calculated for C₃₄H₄₈N₈O₁₀ 728.35, found ES-MS 729.55 [M + M⁺, 751.45 [M + Na]⁺; MALDI-TOF 729.417 [M + H]⁺, 751.3598 [M + Na]⁺; Elemental analysis: calculated for C₃₄H₄₁N₄O₇ C 56.031%, H 6.64%; N 15.38%, found C 56.10%, H 6.60%, N 15.28%.
Compound **23:** ¹H-NMR (CDCl₃) δ 0.87/0.90 (d J 6.59 Hz), 24H), 1.29/1,31 (d J 7.14 Hz), 12H), 1.39 (m, 4H), 2.04 (m, 8H), 3.72 (s, 12H), 3.79 (s, 3H), 3.84 (m, 4H), 4.17 (m, 4H), 4.51 (m, 4H), 5.02 (s, 8H), 5.49 (m, 4H), 6.63 (m, 2H), 6.75 (m, 1H), 6.98 (m, 4H), 7.16 (m, 2H), 7.42 (d J 7.78 Hz), 4H), 7.68 (m, 2H), 8.07 (s, 4H); MS analysis: calculated for C₇₈H₁₀₆N₁₈O₂₂ 1646.77, found MALDI-TOF 1647.730 [M + H]⁺, 1669.732 [M + Na]⁺.
Compound **24:** ¹H-NMR (DMSO-d₆) δ 0.76 (m, 48H), 1.21 (m, 8H), 1.28 (d *J* 7.14 Hz), 24H), 1.98 (m, 16H), 3.58 (overlapping signals 36H), 3.80 (overlapping signals 15H), 4.13 (m, 8H), 4.82 (broad s, 16H), 5.19 (m, 8H), 6.77 (m, 21H), 7.05 (m, 14H), 8.19 (s, 8H); ¹³C-NMR (DMSO-d₆) δ 16.8, 21.7, 22.5, 24.4, 40.5, 48.0, 52.2, 56.0, 66.4, 78.7, 79.1, 99.7, 104.2, 105.2, 106.2, 107.8, 131.8, 136,5, 142.5, 158.4, 159.2, 159.6, 166.0, 166.1, 168.2, 172.7.
Compound **25:** Rt: 19.1 min (C4); MS analysis: calculated for C₁₃₆H₂₀₂N₃₄O₃₆S₂ 2952.872, found MALDI-TOF 2593.310 [M + H]⁺.
Compound **26:** MS analysis: calculated for C₇₀H₈₄N1₆O₁₆ 1404.625, found MALDI-TOF 1427.822 [M + Na]⁺.
Compound **27:** MS analysis: calculated for C₁₅₀H₁₇₈N₃₄O₂₄ 2999.325, found MALDI-TOF 3021.827 [M + H]⁺.
Compound **28:** MS analysis: calculated for C₅₆H₈₆N₂₂O₂₀ 1386.639, found MALDI-TOF 1386.638[M + H]⁺.
Compound **29:** *R*ₜ: 16.78 min (CN); MS analysis: calculated for C₁₂₂H₁₈₂N₄₆O₄₂ 2963.352, found MALDI-TOF 2963.805 [M + H]⁺.
Compound **30:** *R*t: 21.23 min (CN); MS analysis: calculated for C₁₄₆H₁₉₀N₄₆O₃₈ 3195.435, found MALDI-TOF 3195.730 [M + H]⁺.

### Example 5

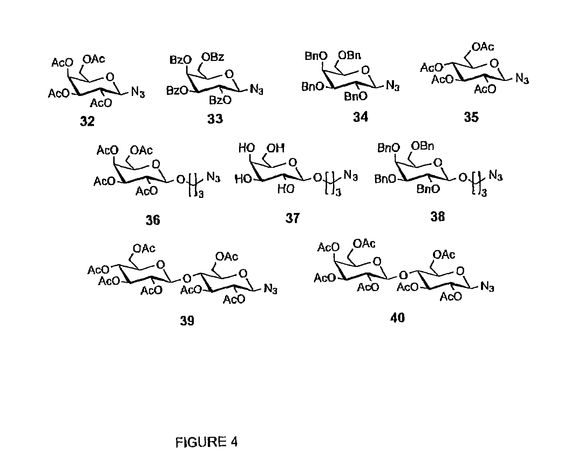

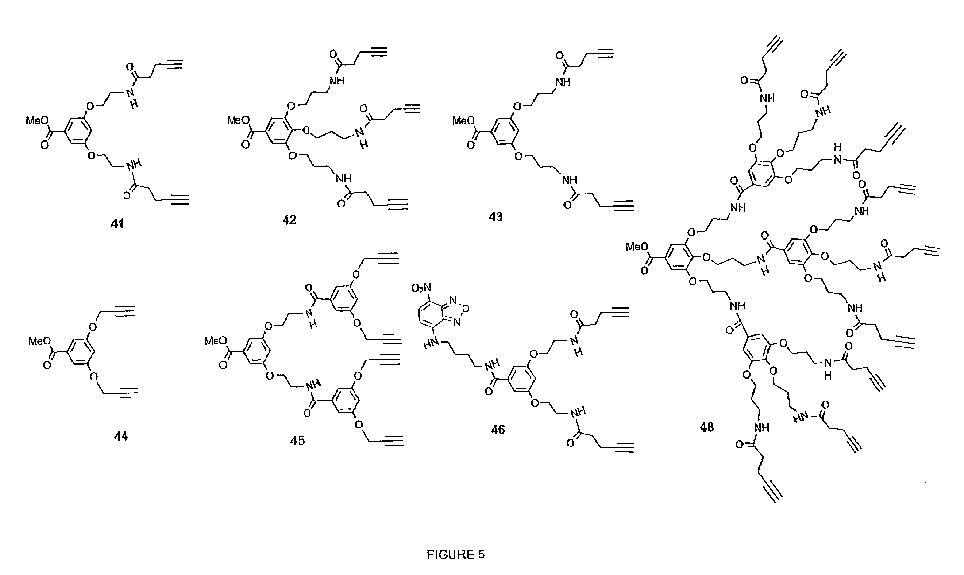

Azido carbohydrates **32-40** of different character, functionalization pattern and reactivity (Fig. 3) were subjected to the cycloaddition reaction with dendrimers **41-47** (Fig. 4) and 19 different triazole glycodendrimers were obtained in good yields.

Furthermore, the use of unprotected carbohydrates was demonstrated as well as the straightforward incorporation of a fluorescent label, relevant for biological evaluation.

The carbohydrate derivatives included the monosaccharide galactose that was used without **32-34,** or with **36-38,** a spacer to the azido function. Its hydroxyl protecting groups were also varied in order to evaluate this parameter. Besides galactose, the monosaccharide glyucose **(35),** the disscharides cellobiose **(39)** and lactose **(40)** were included. The anomeric glycosyl azides were prepared from their respective counterparts by reaction with HBr in AcOH under microwave irradiation. The resulting glycosyl bromides were treated with trimethylsilyl azide and tetrabutyl ammonium fluoride, again using microwave irradiation, which gave the desired peracetylated β-azidosugars for the [3 + 2] cycloaddition reactions in high yields.

Particularly for the **(38)** cellobiose example a solution of peracetylated cellobiose (500 mg), HBr (5 quiv.) and acetic anhydride (4 equiv.) in dry CH₂Cl₂ (10 mL) was exposed to microwave irradiation under sealed vessel conditions at 80 °C for 20 min, after which it was concentrated. The residue was dissolved in dry THF (10 mL) and TMSN₃ (5 equiv.) were added. The mixture was exposed to microwave irradiation at 80 °C for 15 min, then concentrated and purified on a silica column. Elution with toluene-EtOAc 30:1 - 5:1 gave **38** in 99% yield.

The azido carbohydrates were reacted with di- and trivalent first generation dendrimers **(41-44),** one tetravalent second generation dendrimer **(45),** one nonavalent second generation dendrimer **(47),** as well as a divalent dendrimer with an NBD-fluorescent label **(46).** The dendritic main structures were prepared using the approach of Example 1, and these amino acid denderimers were readily synthesized in high yields. Their carbohydrate conjugates were shown to be effective multivalent scaffolds, increasing the binding potencies of their attached carbohydrates by several orders of magnitude.

The copper catalyzed coupling of the azido carbohydrates to divalent alkyne-linked dendrimeric structures involved overnight stirring at room temperature. This yielded a slow conversion to the mono-coupled product. Optimization of the reaction conditions using a microwave reactor (80°C; 20 min.) and CuSO₄ and sodium ascorbate as the copper(I) source led to formation of the desired triazole glycodendrimers in high yields (Table 1, particularly "L").

The use of an excess (1-5 equiv.) of the azido carbohydrate drives the reactions to completion, while the excess could be readily recovered by column chromatography. The relative low product yields in the reactions with unprotected glycosyl azide **36** or fluorescent labeled **46** containing the first generation dendrimer reflects the product purification rather than the coupling efficiency. All coupling products were fully characterized by ¹H, ¹³C-, 2D-COSY NMR and MS.

**Table 1: Microwave assisted copper-(I)-mediated synthesis^{a} of triazole glycodendrimers under microwave conditions.**

| Entry | Azide | Dendrimer | Valency | Yield^{b} |
|---|---|---|---|---|
| 1 | 32 | 41 | 2 | 96 |
| 2 | 32 | 42 | 3 | 95 |
| 3 | 32 | 44 | 2 | 97 |
| 4 | 32 | 45 | 4 | 98 |
| 5 | 33 | 46 | 2 | 68 |
| 6 | 34 | 41 | 2 | 95 |
| 7 | 34 | 44 | 2 | 97 |
| 8 | 35 | 41 | 2 | 97 |
| 9 | 37 | 41 | 2 | 73 |
| 10 | 37 | 42 | 3 | 80 |
| 11 | 37 | 44 | 2 | 94 |
| 12 | 38 | 43 | 2 | 95 |
| 13 | 39 | 41 | 2 | 95 |
| 14 | 40 | 41 | 2 | 95 |
| 15 | 40 | 42 | 3 | 91 |
| 16 | 40 | 44 | 4 | 98 |
| 17 | 40 | 45 | 4 | 91 |
| 18 | 40 | 46 | 2 | 86 |
| 19 | 36 | 47 | 9 | 97 |

| | | | | |
|---|---|---|---|---|
| ^{a} Reagent and conditions: alkyne containing dendrimer (1 equiv.), azido carbohydrate (1,5 equiv. per alkyne), CuSO₄ (0-15 equiv. per alkyne), sodium ascorbate (0.3 equiv. per alkyne) DMF/H₂O, MW 80 °C, 20 min, ^{b} Yield of isolated product. | | | | |

## Claims

1. Multivalent dendrimer, comprising at the dendrimer periphery a number of active compounds, the active compounds being coupled to the dendrimer through a 1,2,3-triazole or 1,2-oxazole heterocyclic bridge.

2. The dendrimer of claim 1, being at least a second generation dendrimer.

3. The dendrimer according to claim 1 or claim 2, being amino acid based.

4. The dendrimer according to any one of the preceding claims, wherein each active compound is selected from the group consisting of peptides, carbohydrates or drugs.

5. The dendrimer according to any one of the preceding claims, wherein the active compounds are the same.

6. The dendrimer according to any one of the preceding claims, wherein the core of the dendrimer is substituted by another active compound than the active compounds at the periphery.

7. Process for the preparation of a multivalent dendrimer according to any one of the preceding claims, comprising reacting a dendrimer having a number of alkyne moieties at its periphery with active compounds comprising an azide or nitrile oxide moiety, or reacting a dendrimer having a number of azide or nitrile oxide moieties at its periphery with active compounds comprising an alkyne moiety.

8. The process of claim 7, wherein the reaction is microwave assisted.

9. The process of claim 7 or claim 8, wherein the alkyne moiety and/or the azide or nitrile oxide moiety is attached to either the dendrimer or the active compound through a spacer.

10. Amino acid based dendrimer having a number of alkyne moieties at its periphery, or having a number of azide or nitrile oxide moieties at its periphery.

11. Use of the multivalent dendrimer according to any one of claims 1 to 6 as a pharmaceutical agent or as a diagnostic agent.
